# EUROPÄISCHE PATENTSCHRIFT

(11) **EP 4 233 710 B1**
(45) Veröffentlichungstag und Bekanntmachung des Hinweises auf die Patenterteilung: **29.05.2024**
(21) Anmeldenummer: 22158801.5
(22) Anmeldetag: 25.02.2022
(51) Int. Cl.: A61B 5/083, A61B 5/08, A61B 5/097

(54) **MESSVORRICHTUNG**
MEASURING DEVICE
DISPOSITIF DE MESURE

(43) Veröffentlichungstag der Anmeldung: 30.08.2023
(73) Patentinhaber: Ventilytics GmbH, 35452 Heuchelheim (DE)
(72) Erfinder: Alexander, Zorn, 35452 Heuchelheim (DE); Steffen, Fuchs, 35633 Lahnau (DE)
(74) Vertreter: Wunderlich & Heim Patentanwälte PartG mbB

(56) Entgegenhaltungen:
- EP-A1- 2 745 775
- DE-U1-202009 013 577
- KR-A- 20030 009 013
- US-A1- 2016 258 920
- US-A1- 2017 184 492
- US-A1- 2018 153 440
- US-A1- 2019 282 124
- US-A1- 2021 052 192

## Beschreibung

Die Erfindung betrifft eine Messvorrichtung zum Ermitteln des CO₂-Gehalts in der Ausatemluft eines Lebewesens wie einer Person oder eines Tiers. Sie weist einen Messraum auf, welcher durch eine Wandung definiert ist. Hierbei kann es sich im Wesentlichen um die Wandung eines Gehäuses und eine weitere Abtrennung handeln. Ferner weist der Messraum eine Lufteinlassöffnung sowie eine Luftauslassöffnung auf. Zum Ermitteln des CO₂-Gehalts ist eine CO₂-Messeinrichtung vorgesehen, welche in Fluidverbindung mit dem Messraum steht. Über diese Fluidverbindung ist ein Gasaustausch zwischen der CO₂-Messeinrichtung und dem Messraum, insbesondere bezüglich der Ausatemluft der Person, möglich, so dass hierüber der CO₂-Gehalt in der Ausatemluft der Person gemessen und ermittelt werden kann, wenn diese durch die Lufteinlassöffnung in Richtung der Luftauslassöffnung ausatmet.

Pulmonale Hypertonie oder Lungenhochdruck bezeichnet eine Erkrankung, bei der der Blutdruck im Lungenkreislauf chronisch erhöht ist. Sie zu diagnostizieren ist nicht ohne weiteres möglich, da es beispielsweise keine Bluttests mit hoher Sensitivität und ausreichend hoher Spezifität gibt. Zurzeit wird die Wahrscheinlichkeit der Diagnose hauptsächlich über eine Echokardiographie abgeschätzt und wenn notwendig mittels Rechtsherzkatheter bestätigt. Hierbei sind jedoch eine hohe Spezialisierung und Kenntnis der die Diagnose stellenden Person notwendig.

Derzeit gibt es Anstrengungen, die Wahrscheinlichkeit der Diagnose zumindest unterstützt durch den aktuellen CO₂-Gehalt in der Ausatemluft zu ermitteln.

Das Messen des CO₂-Gehalts in der Ausatemluft ist bekannt und wird seit langem standardmäßig beispielsweise bei Narkosen durchgeführt. Jedoch sind die entsprechenden Geräte beziehungsweise Einheiten relativ teuer und meist nicht mobil, da sie für den stationären Einsatz in OPs vorgesehen sind.

Grundsätzlich unterscheidet man bei CO₂-Messgeräten zwischen Sidestream- und Main- oder Instream-Messungen. Bei Sidestream-Messungen wird aus dem eigentlichen Luftfluss ein Teil der Strömung mit einem definierten Druck abgezweigt und an oder in diesem die Messung durchgeführt. Hierbei können sehr genaue Messungen erreicht werden. Jedoch sind die Geräte sehr aufwändig und daher sehr hochpreisig.

Bei einer Mainstream-Messung wird die Messung direkt im Luftstrom des Ausatmens durchgeführt. Jedoch sind diese Messungen durch den unterschiedlichen Druckverlauf beim Ausatmen fehlerhaft und weisen Abweichungen von bis zu 50% auf.

Mainstream-Messgeräte sind beispielsweise aus der US 2020/0232971 A1, der US 2002/0029003 A1 oder der US 2018/0078175 A1 bekannt. Diesen ist gemein, dass sie den CO₂-Gehalt in der Ausatemluft ohne Abzweigungen direkt in einer Messröhre messen. Sie haben jedoch den Nachteil, dass dieses Messprinzip deutlich unterschiedliche Messwerte liefert, wodurch eine Auswertung sehr schwierig ist.

Aus der US 2016/258920 A1 eine Messvorrichtung zum Ermitteln eines O2-Gehalts in der Ausatemluft eines Patienten bekannt, mit einem durch eine Wandung definierten Messraum, der eine Lufteinlassöffnung und eine Anzahl von Luftauslassöffnungen aufweist. Die Luftauslassöffnungen sind jeweils durch einen in das Innere des Messraums vorstehenden und zum Messraum hin offenen Zylinder gebildet, in dem jeweils ein Sensor für das Zielgas angeordnet und zur direkten Messung des O₂-Gehalts der Luft in dem Zylinder ausgebildet ist.

Aus der EP 2 745 775 A1 ist eine Messvorrichtung für Ausatemluft eines Patienten bekannt, die ein Substrat aufweist, in dem in einem Hohlraum ein O₂-Sensor bzw. ein Drucksensor angeordnet ist und ein zylindrischer Vorsprung an dem Hohlraum so vorgesehen ist, dass er sich von dem Substrat weg in einen Messraum erstreckt, wo er die Atemluftströmung stört. Der jeweilige Sensor erfasst die gestörte Atemluft-Strömungsrate.

Aus der US 2018/153440 A1 ist ein Verfahren zum Ermitteln des CO₂-Gehalts in der Ausatemluft eines Lebewesens mit einer Messvorrichtung bekannt, die einen Messraum mit Lufteinlassöffnung und Luftauslassöffnung und einer CO₂-Messeinrichtung in Fluidverbindung mit dem Messraum aufweist, wobei zur Verbesserung der Genauigkeit über mehrere Atemzyklen gemessen wird.

Der Erfindung liegt daher die A u f g a b e zugrunde, eine Messvorrichtung zum Ermitteln des CO₂-Gehalts in der Ausatemluft eines Lebewesens, welche einfach aufgebaut ist und dennoch eine hohe Messgenauigkeit hat, sowie ein Verfahren zum Ermitteln des CO₂-Gehalts mit einer solchen Messvorrichtung anzugeben.

Diese Aufgabe wird erfindungsgemäß durch eine Messvorrichtung mit den Merkmalen des Anspruchs 1 sowie mit einem Verfahren zum Ermitteln des CO₂-Gehalts in der Ausatemluft eines Lebewesens mit den Merkmalen des Anspruchs 9 gelöst.

Bevorzugte Weiterbildungen und Ausführungsformen der Erfindung sind in den Unteransprüchen angegeben. Die erfindungsgemäße Messvorrichtung ist dadurch weitergebildet, dass sie einen Sensortopf aufweist, welcher sich zumindest teilweise in den Messraum erstreckt. Hierbei ist vorgesehen, dass sich der Sensortopf durch die Wandung des Messraumes in das Innere des Messraumes erstreckt, wobei der Durchmesser oder die lichte Weite des Messraumes im Bereich des Sensortopfes verringert ist. Der Sensortopf ist zum Messraum im Wesentlichen offen ausgebildet. Dies bedeutet, dass Ausatemluft in den Sensortopf eindringen kann. Ferner ist die CO₂-Messeinrichtung in dem Sensortopf angeordnet und zur direkten Messung des CO₂-Gehalts der Luft in dem Sensortopf ausgebildet.

Des Weiteren betrifft die Erfindung ein Verfahren zum Ermitteln des CO₂-Gehalts in der Ausatemluft eines Lebewesens mit einer erfindungsgemäßen Messvorrichtung, wobei das Lebewesen mehrfach durch den Messraum der Messvorrichtung ausatmet.

Der Erfindung liegt die Erkenntnis zugrunde, dass es ein großes Problem bei gattungsgemäßen Messvorrichtungen ist, dass diese ungenaue Messergebnisse durch die im Ausatmen vorhandene Änderung des Drucks erzeugen. Entsprechend der Erfindung wurde erkannt, dass die Variation des Druckes durch das Vorsehen eines Sensortopfes, welcher in Richtung des Messraumes geöffnet ist und zumindest teilweise in diesen hineinragt, reduziert werden kann. Erstaunlicherweise sind die Druckbedingungen beim Ausatmen durch den Messraum innerhalb des Sensortopfes deutlich konstanter als innerhalb des restlichen Bereiches des Messraumes.

Ein weiterer Grundgedanke der Erfindung ist, dass der Sensortopf zum Messraum hin im Wesentlichen offen ausgebildet ist. Dies kann beispielsweise bedeuteten, dass es keine Verringerung des Durchmessers beziehungsweise des Öffnungsdurchmessers des Sensortopfes zum Messraum hingibt. Jedoch kann auch vorgesehen sein, einen Filter, welcher eine sehr gute Luftdurchströmung, also einen geringen Luftwiderstand, aufweist, im oder am oberen Ende des Sensortopfes vorzusehen, um die CO₂-Messeinrichtung vor Bakterien oder Verschmutzungen zu schützen.

Bei dem Lebewesen kann es sich sowohl um einen Menschen, wie auch um ein Tier, insbesondere um ein Säugetier, beispielsweise ein Pferd, handeln.

Der Sensor- oder Messtopf selbst kann im Wesentlichen zylinderartig ausgeformt sein, wobei eine Stirnfläche als Öffnung ausgebildet ist. Bevorzugt ist es, wenn der Messraum einen konstanten Durchmesser über seine gesamte Länge von der Lufteinlassöffnung zu einer Luftauslassöffnung aufweist. Dieser Durchmesser wird durch den Sensortopf in einem Bereich verringert. Durch das Reduzieren des Durchmessers entstehen Luftwirbel in diesem Bereich, so dass sichergestellt wird, dass das sich im Sensortopf befindliche Gas im Wesentlichen der aktuellen Ausatemluft entspricht. Hierdurch wird eine hohe Messgenauigkeit erreicht.

Erfindungsgemäß ist das Verhältnis des Volumens des Messraumes, ohne Berücksichtigung des Sensortopfes, zum Volumen des Sensortopfes im Bereich von 7 : 1 bis 13 : 1. Idealerweise liegt es im Bereich von 10 : 1. Durch dieses Verhältnis wird erstaunlicherweise erreicht, dass während des Ausatmens durch den Messraum ein relativ konstanter Druck im Sensortopf vorhanden ist, so dass eine hochgenaue Messung des dort vorhandenen CO₂-Gehalts in Gas durchgeführt werden kann.

Bevorzugt ist es, wenn die Lufteinlassöffnung und die Luftauslassöffnung an gegenüberliegenden Enden des Messraumes vorgesehen sind und ihre Verlängerung eine im Wesentlichen gerade virtuelle Messröhre ausbilden. Anders ausgedrückt ist es bevorzugt, wenn die Lufteinlassöffnung und die Luftauslassöffnung etwa denselben Durchmesser beziehungsweise dieselbe Öffnungsfläche aufweisen und in einer geraden Position zueinander platziert sind. Somit kann durch den Messraum eine virtuelle Messröhre mit einem konstanten Durchmesser ausgebildet werden.

Grundsätzlich kann der Sensortopf beliebig weit in den Messraum beziehungsweise in die virtuelle Messröhre hineinragen. Bevorzugt ist es jedoch, wenn der Sensortopf bis zu mindestens einem Drittel des Durchmessers und maximal zwei Drittel des Durchmessers der virtuellen Messröhre in den Messraum hineinragend ausgebildet ist. Bevorzugt ist es hierbei, wenn er ungefähr die Hälfte des Durchmessers der virtuellen Messröhre einnimmt. Anders ausgedrückt, verringert der Sensortopf, der in die virtuelle Messröhre hineinragt, in diesem Bereich den Durchmesser der virtuellen Messröhre zwischen einem Drittel und zwei Dritteln. Diese Dimensionierung sorgt zum einen für eine gute Belüftung des Sensortopfes selbst und zum anderen wird dadurch auch ein relativ konstanter Druck im Sensortopf erreicht.

Die hohe Messgenauigkeit kann weiter verbessert werden, wenn der Sensortopf im Wesentlichen senkrecht zu einer Längsachse des Messraums und/oder der virtuellen Messröhre ausgebildet ist. Anders ausgedrückt, liegt die Öffnung des Sensortopfes parallel zu einer Ebene, welche durch die Rotationsachse der virtuellen Messröhre verläuft. Grundsätzlich ist auch ein Abkippen in Richtung der Lufteinlassöffnung oder der Luftauslassöffnung möglich, jedoch wird mit einer Dimensionierung, welche dies nicht so ausführt, ein besseres Messergebnis erreicht.

Generell können verschiedene Sensoren in der CO₂-Messeinrichtung vorgesehen sein. Bevorzugt ist jedoch entsprechend der Erfindung ein infrarotbasierter Sensor, da dieser beispielsweise über den gesamten Innenraum des Sensortopfes seine Messung durchführen kann.

Hierbei hat es sich als vorteilhaft für die Messgenauigkeit herausgestellt, wenn die CO₂-Messeinrichtung zum Durchführen einer kontinuierlichen Messung ausgebildet ist. Somit können während des Ausatmens mehrere Messwerte ermittelt werden, die anschließend weiterverarbeitet werden können. Grundsätzlich ist aber auch eine diskontinuierliche Messung möglich, die mit geringen Abständen durchgeführt wird.

Bis auf den Sensortopf kann das Innere des Messraums bevorzugt so ausgebildet sein, dass die Luft im Wesentlichen verwirbelungsarm oder sogar -los von der Lufteinlassöffnung zur Luftauslassöffnung strömen kann. Dies führt wiederum zu einem besseren Luftstrom, so dass der Druck, der sich im Sensortopf einstellt, ausreichend konstant ist. Allgemein ist es aber auch möglich, Ableitelemente vorzusehen, die jedoch eine genaue Platzierung und Dimensionierung erfordern. Durch die vorgeschlagene Konstruktion einer im Wesentlichen verwirbelungsarmen Luftführung wird die Gesamtkonstruktion erleichtert. Daher ist der Messraum bevorzugt im Wesentlichen gerade ausgebildet, wobei grundsätzlich auch eine leichte Biegung möglich wäre. Der Messraum kann hierzu beispielsweise einen U-ähnlichen oder U-förmigen Querschnitt ausweisen.

Die Erfindung betrifft ferner ein Verfahren zum Ermitteln des CO₂-Gehalts in der Ausatemluft eines Lebewesens mit einer erfindungsgemäßen Messeinrichtung. Hierbei ist vorgesehen, dass das Lebewesen mehrfach durch den Messraum ausatmet. Während dieses Ausatmens können Messungen mittels der CO₂-Messeinrichtung der erfindungsgemäßen Messvorrichtung durchgeführt werden.

Hierbei ist es bevorzugt, wenn verhindert wird, dass das Lebewesen parallel durch die Nase ausatmet. Dies kann beispielsweise durch eine Nasenklammer oder ein Zuhalten der Nase erreicht werden.

Es ist bevorzugt, wenn die CO₂-Messeinrichtung eine kontinuierliche Messung des CO₂-Gehalts in der Ausatemluft beziehungsweise von dem Gas innerhalb des Sensortopfes durchführt. Auf diese Weise kann der optimale Zeitpunkt ermittelt werden, zu dem die CO₂-Messung ein zur Weiterverarbeitung geeignetes Ergebnis liefert.

Es können mehrere, mindestens 7, bevorzugt mehr als 9 von der CO₂-Messeinrichtung ermittelte Maximalwerte innerhalb eines Zeitbereiches gespeichert werden. Der Zeitbereich kann beispielsweise ein Ausatemvorgang sein. Ein Maximalwert kann hierbei der höchste Wert innerhalb eines Messintervalls sein. Es kann beispielsweise vorgesehen sein, dass während des Ausatmens der CO2-Gehalt der Luft oder des Gases im Sensortopf kontinuierlich gemessen wird. Dieser steigt zunächst an, fällt dann, sobald das Ausatmen endet, wiederum ab. Ein derartiges lokales Maximum kann auch als Maximalwert herangezogen werden. Es kann so beispielsweise der höchste vorliegende CO₂ Wert in einer Ausatmung ermittelt werden.

Um die Auswertegenauigkeit weiter zu erhöhen, können zur Endauswertung aus einer Gruppe von Maximalwerten eine gewisse Anzahl - beispielsweise 2 oder 3 der niedrigsten (geringsten) Maximalwerte - nicht berücksichtigt werden. Auf diese Weise können in einfacher Art Fehlmessungen beziehungsweise Messungen mit einer niedrigen Genauigkeit herausgefiltert werden.

Bevorzugt ist es, wenn als Ausgabewert des gemessenen CO₂s ein, insbesondere arithmetischer, Mittelwert oder Maximalwert ausgegeben wird. Diese Werte können auch bereinigt sein, bevor der entsprechende Mittelwert errechnet wird.

Ferner kann es vorgesehen sein, dass die von der CO₂-Messeinrichtung ermittelten Messwerte mit einem Korrekturfaktor versehen werden, um konstruktionsbedingte Abweichungen, die konstant sind, zu berücksichtigen.

Die Erfindung wird nachfolgend anhand eines Ausführungsbeispiels und schematischen Zeichnungen näher erläutert. In diesen Zeichnungen zeigen:
- Fig. 1: eine Seitenansicht einer erfindungsgemäßen Messvorrichtung;
- Fig. 2: eine Seitenansicht der erfindungsgemäßen Messvorrichtung mit teilweise entfernten Seiten;
- Figuren 3 und 4: zwei perspektivische Seitenansichten der erfindungsgemäßen Messvorrichtung; und
- Fig. 5: die erfindungsgemäße Messvorrichtung aus Fig. 4 mit teilweise entfernten Seiten.

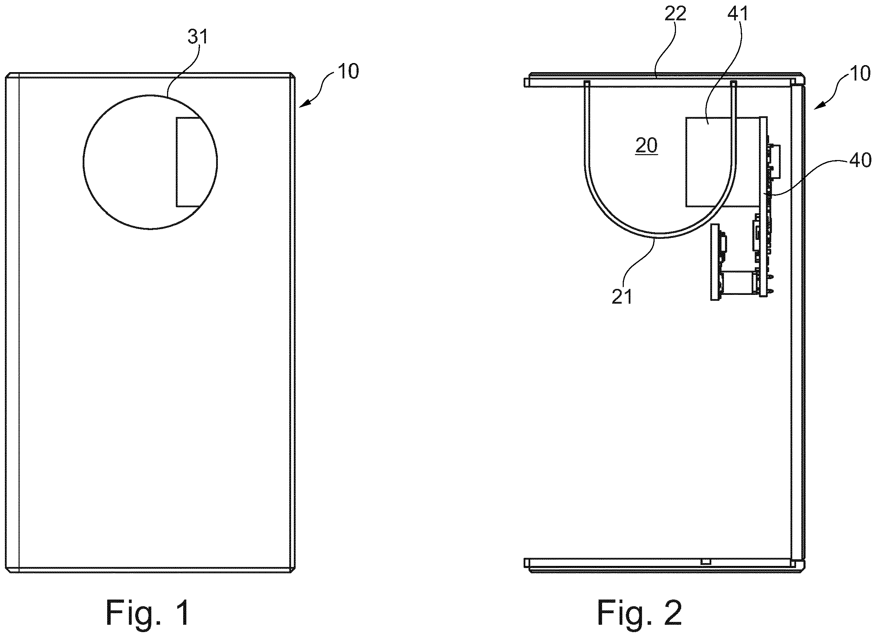

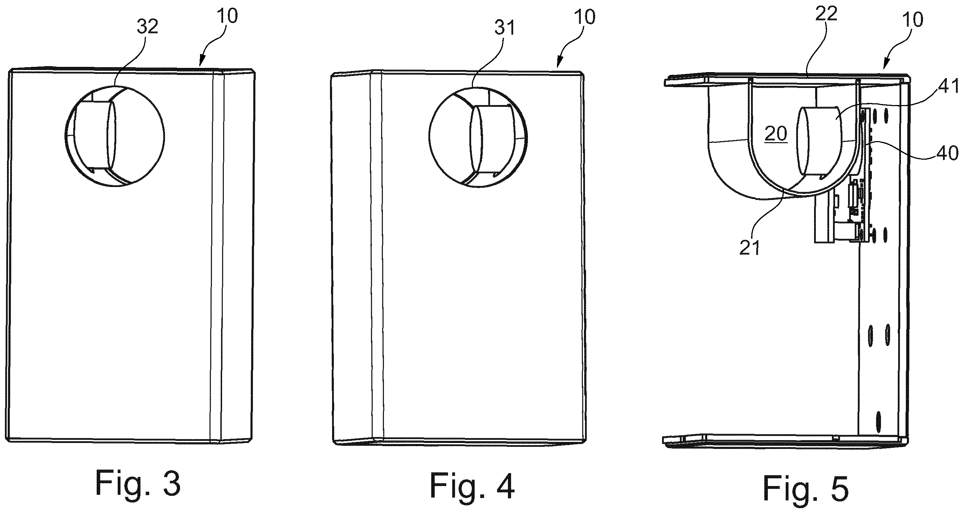

Im Folgenden wird eine erfindungsgemäße Messvorrichtung 10 basierend auf den Figuren näher erläutert. Hierbei zeigt Fig. 1 eine seitliche Ansicht einer erfindungsgemäßen Messvorrichtung 10. In Fig. 2 ist dieselbe Ansicht der erfindungsgemäßen Messvorrichtung 10 dargestellt, wobei drei Seitenwände entfernt wurden, damit das Innere sichtbar ist. Figuren 3 und 4 zeigen jeweils zwei leicht perspektivische Ansichten von unterschiedlichen Seiten der erfindungsgemäßen Messvorrichtung 10. Abschließend ist in Fig. 5 die Messvorrichtung 10 entsprechend Fig. 4 dargestellt, wobei wiederum drei Seiten entfernt wurden, damit ein Teil des Inneren der Messvorrichtung 10 sichtbar ist.

Die hier dargestellte Messvorrichtung 10 weist ein im Wesentlichen quaderförmiges Gehäuse auf. Die Erfindung ist jedoch nicht auf diese Form des Gehäuses eingeschränkt. Ferner ist eine Lufteinlassöffnung 31 und eine Luftauslassöffnung 32 vorgesehen. Beim Verwenden der Messvorrichtung 10 wird durch die Lufteinlassöffnung 31 durch einen Messraum, welcher auch als Messkammer 20 bezeichnet wird, ausgeatmet. Grundsätzlich können die Lufteinlassöffnung 31 und die Luftauslassöffnung 32 auch vertauscht sein.

Insbesondere in Figuren 4 und 5 ist der Messraum 20 gezeigt. Er wird durch eine U-förmige Wandung 21 sowie einen Teil der Außenwandung 22 ausgebildet. Wie sich aus Figuren 1, 3 und 4 ergibt, führen zum Messraum 20 die Lufteinlassöffnung 31 und die Luftauslassöffnung 32, die in dieser Ausführungsform kreisrund ausgebildet sind. Es sind aber auch andere Öffnungsquerschnitte möglich. Bevorzugt sind die Öffnungen, wie insbesondere aus Fig. 1 hervorgeht, genau gegenüberliegend ausgebildet, so dass ihre Verlängerung eine virtuelle Messröhre ausbildet.

In den Messraum 20 erstreckt sich eine CO₂-Messeinrichtung 40 hinein. Diese weist einen Sensortopf 41 auf, welcher in dem Messraum 20 hineinragt. In dem Sensortopf 41 befindet sich eine CO₂-Messeinrichtung 40, die beispielsweise mittels einer Infrarotmessung das im Sensortopf 41 befindliche CO₂ misst. Bevorzugt ist der Sensortopf gleich weit von der der Lufteinlassöffnung 31 und der Luftauslassöffnung 32 entfernt und befindet sich in der Mitte zwischen ihnen.

Der Sensortopf 41 ist in einer Fluidverbindung mit dem Messraum 20. Er kann einfach offen ausgelegt sein. Bevorzugt ist es jedoch, wenn er einen Filter oder eine Membran aufweist, um Verschmutzungen des Innenraums des Sensortopfes 41 zu verhindern. Wesentlich ist hierbei, dass dieser/diese eine gute Luftdurchlässigkeit aufweist.

Wie insbesondere aus Fig. 1 ersichtlich, ragt der Sensortopf 41 der CO₂-Messeinrichtung 40 in eine virtuelle Röhre, die zwischen der Lufteinlassöffnung 31 und der Luftauslassöffnung 32 ausgebildet ist.

Entsprechend der Erfindung wurde erkannt, dass beim Ausatmen durch die Lufteinlassöffnung 31 sich innerhalb des Sensortopfes 41 ein relativ konstanter Druck einstellt, so dass die dort vorhandene Messung unerwarteterweise eine sehr hohe Genauigkeit aufweist.

## Patentansprüche

1. Messvorrichtung (10) zum Ermitteln des CO₂-Gehalts in der Ausatemluft eines Lebewesens,
mit einem Messraum (20), welcher durch eine Wandung definiert ist, wobei der Messraum (20) eine Lufteinlassöffnung (31) und eine Luftauslassöffnung (32) aufweist,
mit einer CO₂-Messeinrichtung (40), welche in Fluidverbindung mit dem Messraum (20) steht,
wobei ein Sensortopf (41), welcher sich zumindest teilweise in den Messraum (20) erstreckt, vorgesehen ist,
wobei sich der Sensortopf (41) durch die Wandung (21, 22) des Messraumes (20) in das Innere des Messraumes (20) erstreckt, wobei der Durchmesser des Messraums (20) im Bereich des Sensortopfes (41) verringert ist,
wobei der Sensortopf (41) zum Messraum (20) hin im Wesentlich offen ausgebildet ist,
wobei die CO₂-Messeinrichtung (40) in dem Sensortopf (41) angeordnet ist und zur direkten Messung des CO₂-Gehalts der Luft in dem Sensortopf (41) ausgebildet ist, und
wobei das Verhältnis des Volumens des Messraumes (20) ohne Berücksichtigung des Sensortopfes (41) zum Volumen des Sensortopfes (41) im Bereich von 7 : 1 bis 13 : 1 liegt.

2. Messvorrichtung (10) nach Anspruch 1,
**dadurch gekennzeichnet,**
**dass** der Messraum (20) einen konstanten Durchmesser über seine gesamte Länge vom der Lufteinlassöffnung (31) zur Luftauslassöffnung (32) aufweist, welcher durch den Sensortopf (41) in einem Bereich verringert ist.

3. Messvorrichtung (10) nach Anspruch 1 oder 2,
**dadurch gekennzeichnet,**
**dass** die Lufteinlassöffnung (31) und die Luftauslassöffnung (32) an gegenüberliegenden Enden des Messraumes (20) vorgesehen sehen sind und ihre Verlängerung eine gerade virtuelle Messröhre ausbildet.

4. Messvorrichtung (10) nach Anspruch 3,
**dadurch gekennzeichnet,**
**dass** der Sensortopf (41) im Wesentlichen senkrecht zu einer Längsachse der virtuellen Messröhre angeordnet ist.

5. Messvorrichtung (10) nach einem der Ansprüche 1 bis 4,
**dadurch gekennzeichnet,**
**dass** ein infrarotbasierter Sensor in der CO₂-Messeinrichtung (40) vorgesehen ist.

6. Messvorrichtung (10) nach einem der Ansprüche 1 bis 5,
**dadurch gekennzeichnet**
**dass** die CO₂-Messeinrichtung (40) zum Durchführen einer kontinuierlichen Messung ausgebildet ist.

7. Messvorrichtung (10) nach einem der Ansprüche 1 bis 6,
**dadurch gekennzeichnet,**
**dass** das Innere des Messraumes (20) derart ausgebildet ist, dass Luft bis auf den Sensortopf (41) im Wesentlichen verwirbelungslos von der Lufteinlassöffnung (31) zur Luftauslassöffnung (32) strömen kann.

8. Messvorrichtung (10) nach einem der Ansprüche 1 bis 7,
**dadurch gekennzeichnet,**
**dass** der Messraum (20) einen U-förmigen Querschnitt aufweist,
wobei der Messraum (20) durch eine U-förmige Wandung (21) sowie einen Teil der Außenwandung (22) ausgebildet ist, und
wobei die U-förmige Wandung (21) an die Außenwandung (22) anschließt, sodass ein der Außenwandung (22) gegenüberliegender Teil der U-förmigen Wandung (21) zum Messraum (20) hin konkav ausgestaltet ist.

9. Verfahren zum Ermitteln des CO₂-Gehalts in der Ausatemluft eines Lebewesens, mit einer Messvorrichtung (10) nach einem der Ansprüche 1 bis 8, wobei das Lebewesen mehrfach in die Lufteinlassöffnung (31) durch den Messraum (20) ausatmet.

10. Verfahren nach Anspruch 9,
**dadurch gekennzeichnet,**
**dass** die CO₂-Messeinrichtung (40) eine kontinuierliche Messung des CO₂-Gehalts durchführt.

11. Verfahren nach Anspruch 9 oder 10,
**dadurch gekennzeichnet**
**dass** mindestens 7 von der CO₂-Messeinrichtung (40) ermittelte CO₂-Maximalwerte gespeichert werden.

12. Verfahren nach einem der Ansprüche 9 bis 11,
**dadurch gekennzeichnet,**
**dass** zur Endauswertung mindestens die zwei niedrigsten CO₂-Maximalwerte von mehreren CO₂-Maximalwerten nicht berücksichtigt werden.

13. Verfahren nach einem der Ansprüche 9 bis 12,
**dadurch gekennzeichnet,**
**dass** als Ausgabewert ein, insbesondere arithmetisches, Mittel mehrerer Maximalwerte ausgegeben wird.

14. Verfahren nach einem der Ansprüche 9 bis 13,
**dadurch gekennzeichnet,**
**dass** die von der CO₂-Messeinrichtung (40) ermittelten Messwerte mit einem Korrekturfaktor versehen werden.

## Claims

1. Measuring apparatus (10) for determining the CO₂ content in the exhaled air of a living being,
having a measuring space (20), which is defined by a wall, wherein the measuring space (20) comprises an air inlet opening (31) and an air outlet opening (32),
having a CO₂-measuring device (40), which is in fluidic communication with the measuring space (20),
**wherein** a sensor pot (41), which extends at least in part into the measuring space (20), is provided,
**wherein** the sensor pot (41) extends through the wall (21, 22) of the measuring space (20) into the interior of the measuring space (20), wherein the diameter of the measuring space (20) is reduced in the region of the sensor pot (41),
w h e re i n the sensor pot (41) is configured so as to be substantially open towards the measuring space (20),
w here i n the CO₂-measuring device (40) is arranged in the sensor pot (41) and is configured to measure the CO₂ content of the air in the sensor pot (41) directly, and
wherein the ratio of the volume of the measuring space (20), disregarding the sensor pot (41), to the volume of the sensor pot (41) is in the range of from 7:1 to 13:1.

2. Measuring apparatus (10) according to claim 1,
**characterized in that**
the measuring space (20) has a constant diameter over its entire length from the air inlet opening (31) to the air outlet opening (32), which diameter is reduced in one region by the sensor pot (41).

3. Measuring apparatus (10) according to claim 1 or 2,
**characterized in that**
the air inlet opening (31) and the air outlet opening (32) are provided at opposite ends of the measuring space (20), and their prolongation forms a straight virtual measuring tube.

4. Measuring apparatus (10) according to claim 3,
**characterized in that**
the sensor pot (41) is arranged substantially perpendicular to a longitudinal axis of the virtual measuring tube.

5. Measuring apparatus (10) according to any one of claims 1 to 4,
**characterized in that**
an infrared-based sensor is provided in the CO₂-measuring device (40).

6. Measuring apparatus (10) according to any one of claims 1 to 5,
**characterized in that**
the CO₂-measuring device (40) is configured to carry out a continuous measurement.

7. Measuring apparatus (10) according to any one of claims 1 to 6,
**characterized in that**
the interior of the measuring space (20) is configured so that air, apart from the sensor pot (41), is able to flow from the air inlet opening (31) to the air outlet opening (32) substantially without turbulence.

8. Measuring apparatus (10) according to any one of claims 1 to 7,
**characterized in that**
the measuring space (20) has a U-shaped cross section,
wherein the measuring space (20) is formed by a U-shaped wall (21) and part of the outer wall (22), and
wherein the U-shaped wall (21) adjoins the outer wall (22), so that a part of the U-shaped wall (21) opposite the outer wall (22) is designed to be concave towards the measuring space (20).

9. Method for determining the CO₂ content in the exhaled air of a living being, having a measuring apparatus (10) according to any one of claims 1 to 8, wherein the living being exhales into the air inlet opening (31) through the measuring space (20) multiple times.

10. Method according to claim 9,
**characterized in that**
the CO₂-measuring device (40) carries out a continuous measurement of the CO₂ content.

11. Method according to claim 9 or 10,
**characterized in that**
at least 7 maximum CO2-values determined by the CO₂-measuring device (40) are stored.

12. Method according to any one of claims 9 to 11,
**characterized in that**
at least the two lowest maximum CO2-values of a plurality of maximum CO2-values are disregarded in the final evaluation.

13. Method according to any one of claims 9 to 12,
**characterized in that**
there is outputted as the output value a mean, in particular an arithmetic mean, of a plurality of maximum values.

14. Method according to any one of claims 9 to 13,
**characterized in that**
the measured values determined by the CO₂-measuring device (40) are provided with a correction factor.

## Revendications

1. Dispositif de mesure (10) pour déterminer la teneur en CO₂ dans l'air expiré d'un être vivant,
avec un espace de mesure (20) qui est défini par une paroi, l'espace de mesure (20) présentant une ouverture d'entrée d'air (31) et une ouverture de sortie d'air (32),
avec un dispositif de mesure du CO₂ (40), qui est en communication fluidique avec l'espace de mesure (20),
un capteur en forme de pot (41) étant prévu, qui s'étend au moins partiellement dans l'espace de mesure (20),
le capteur en forme de pot (41) s'étendant à travers la paroi (21, 22) de l'espace de mesure (20) à l'intérieur de l'espace de mesure (20), le diamètre de l'espace de mesure (20) étant réduit dans la zone du capteur en forme de pot (41),
le capteur en forme de pot (41) étant réalisé sensiblement ouvert vers l'espace de mesure (20),
le dispositif de mesure de CO₂ (40) étant disposé dans le capteur en forme de pot (41) et étant conçu pour mesurer directement la teneur en CO₂ de l'air dans le capteur en forme de pot (41), et
le rapport entre le volume de l'espace de mesure (20), sans prise en compte du capteur en forme de pot (41), et le volume du capteur en forme de pot (41) se situant dans la plage de 7 : 1 à 13 : 1.

2. Dispositif de mesure (10) selon la revendication 1,
**caractérisé en ce que**
l'espace de mesure (20) a un diamètre constant sur toute sa longueur depuis l'ouverture d'entrée d'air (31) jusqu'à l'ouverture de sortie d'air (32), lequel est réduit dans une zone par le capteur en forme de pot (41).

3. Dispositif de mesure (10) selon la revendication 1 ou 2,
**caractérisé en ce que**
l'ouverture d'entrée d'air (31) et l'ouverture de sortie d'air (32) sont prévues aux extrémités opposées de l'espace de mesure (20) et leur prolongement forme un tube de mesure virtuel droit.

4. Dispositif de mesure (10) selon la revendication 3,
**caractérisé en ce que**
le capteur en forme de pot (41) est disposé sensiblement perpendiculairement à un axe longitudinal du tube de mesure virtuel.

5. Dispositif de mesure (10) selon l'une des revendications 1 à 4,
**caractérisé en ce que**
un capteur basé sur l'infrarouge est prévu dans le dispositif de mesure du CO₂ (40).

6. Dispositif de mesure (10) selon l'une des revendications 1 à 5,
**caractérisé en ce que**
le dispositif de mesure de CO₂ (40) est conçu pour effectuer une mesure en continu.

7. Dispositif de mesure (10) selon l'une des revendications 1 à 6,
**caractérisé en ce que**
l'intérieur du compartiment de mesure (20) est conçu de telle sorte que l'air, à l'exception du pot de capteur (41), peut s'écouler sensiblement sans tourbillon de l'ouverture d'entrée d'air (31) à l'ouverture de sortie d'air (32).

8. Dispositif de mesure (10) selon l'une des revendications 1 à 7,
**caractérisé en ce que**
l'espace de mesure (20) présente une section transversale en forme de U, l'espace de mesure (20) est formé par une paroi en forme de U (21) ainsi que par une partie de la paroi extérieure (22), et
la paroi en forme de U (21) se raccorde à la paroi extérieure (22), de sorte qu'une partie de la paroi en forme de U (21) opposée à la paroi extérieure (22) est réalisée de manière concave vers l'espace de mesure (20).

9. Procédé pour déterminer la teneur en CO₂ de l'air expiré par un être vivant, avec un dispositif de mesure (10) selon l'une des revendications 1 à 8, dans lequel l'être vivant expire plusieurs fois dans l'ouverture d'entrée d'air (31) à travers l'espace de mesure (20).

10. Procédé selon la revendication 9,
**caractérisé en ce que**
le dispositif de mesure de CO₂ (40) effectue une mesure continue de la teneur en CO₂.

11. Procédé selon la revendication 9 ou 10,
**caractérisé en ce que**
au moins 7 valeurs maximales de CO₂ déterminées par le dispositif de mesure de CO₂ (40) sont enregistrées.

12. Procédé selon l'une des revendications 9 à 11,
**caractérisé en ce que**
pour l'évaluation finale, au moins les deux valeurs maximales de CO₂ les plus basses parmi plusieurs valeurs maximales de CO₂ ne sont pas prises en compte.

13. Procédé selon l'une des revendications 9 à 12,
**caractérisé en ce que**
une moyenne, en particulier arithmétique, de plusieurs valeurs maximales est émise comme valeur de sortie.

14. Procédé selon l'une des revendications 9 à 13
**caractérisé en ce que**
les valeurs de mesure déterminées par le dispositif de mesure de CO₂ (40) sont munies d'un facteur de correction.
